# EUROPEAN PATENT APPLICATION

(11) **EP 3 593 714 A1**
(43) Date of publication of application: **15.01.2020**
(21) Application number: 18764539.5
(22) Date of filing: 26.02.2018
(51) Int. Cl.: A61B 5/0408, A61B 5/0402, A61B 5/0476, A61B 5/0488, A61B 5/05

(54) **ELECTRODE FOR BIOLOGICAL INFORMATION MEASUREMENT AND METHOD FOR PRODUCING ELECTRODE FOR BIOLOGICAL INFORMATION MEASUREMENT**

(30) Priority: 07.03.2017 JP 2017042355
(71) Applicant: Alps Alpine Co., Ltd., Ota-ku, Tokyo 1458501 (JP)
(72) Inventor: SUZUKI, Tsuyoshi, Tokyo 145-8501 (JP); AKIYAMA, Toshinori, Tokyo 145-8501 (JP)
(74) Representative: Schmitt-Nilson Schraud Waibel Wohlfrom Patentanwälte Partnerschaft mbB
(86) International application number: PCT/JP2018/006921
(87) International publication number: WO 2018/163881

(57) **Abstract**

[Object] To provide a vital sign information measuring electrode that can measure vital sign information stably without using an electrically conductive gel.

[Solution] A vital sign information measuring electrode 1 includes a substrate 10 and an electrode layer 20 disposed on a surface of the substrate 10, a part of the electrode layer serving as a contact portion that can come into contact with a living body. The contact portion 21 of the electrode layer 20 has a dispersion structure in which silver chloride and silver or a silver alloy are dispersed in a synthetic resin 52. In the contact portion 21 of the electrode layer 20, the content of silver chloride on a surface 21S side on which the contact portion 21 comes into contact with the living body is higher than the content of silver chloride on an inner side.

## Description

### Technical Field

The present invention relates to a vital sign information measuring electrode that is an electrode for measuring vital sign information such as brain waves and to a method for producing the vital sign information measuring electrode.

### Background Art

In recent years, there is an increasing need for measuring vital sign information such as pulse waves, cardioelectric potential, myoelectric potential, body mass index, and brain waves as electric signals for the purpose of health care, observation of the condition of a patient, etc. In the measurement, to stabilize the contact between the living body and an electrode for measuring the vital sign information (a vital sign information measuring electrode), an electrically conductive gel is generally used between the vital sign information measuring electrode and the living body. However, since the electrically conductive gel is highly viscous, its handleability during application is poor, and a troublesome wiping operation is necessary after the measurement. There is therefore a need for a vital sign information measuring electrode that does not require use of the electrically conductive gel.

PTL 1 describes an example of the vital sign information measuring electrode that can meet the need. In this electrode, a lead terminal is insert-molded in a molded product of electrically conductive carbon. A coating layer containing silver and/or silver chloride is present on the surface of the lead terminal, and a topcoat layer is provided as needed.

### Citation List

### Patent Literature

PTL 1: Japanese Unexamined Patent Application Publication No. 9-168518

### Summary of Invention

### Technical Problem

However, in practice, even when the vital sign information measuring electrode described in PTL 1 is used, if the electrode is brought into direct contact with a living body for measurement, temporal drift of the measurement voltage occurs, so it is difficult to maintain measurement stability. It is therefore necessary to apply the electrically conductive gel between the vital sign information measuring electrode and the living body. Even when the electrically conductive gel is used, the voltage drift occurs in some cases, so that it is necessary to wait until the voltage is stabilized and the measurement can be performed.

The present invention has been made in view of the foregoing circumstances, and it is an object to provide a vital sign information measuring electrode that can measure vital sign information without using the electrically conductive gel and to provide a method for producing the vital sign information measuring electrode.

### Solution to Problem

To solve the foregoing problem, one aspect of the present invention provides a vital sign information measuring electrode including: a substrate; and an electrode layer disposed on a surface of the substrate, a part of the electrode layer serving as a contact portion that can come into contact with a living body. The contact portion of the electrode layer has a dispersion structure in which silver chloride and silver or a silver alloy are dispersed in a synthetic resin. In the contact portion of the electrode layer, the content of silver chloride on a surface side on which the contact portion comes into contact with the living body is higher than the content of silver chloride on an inner side.

The vital sign information measuring electrode is a device that has a part (contact portion) coming into contact with a living body to collect vital sign information as an electric signal and then outputs the electric signal to the outside. In the electrode layer of the vital sign information measuring electrode, charge transfer in the portion in contact with the living body is effected by ions (chloride ions), and therefore silver chloride is suitable for the portion coming into contact with the living body. In the contact portion of the electrode layer of the vital sign information measuring electrode according to the present invention, the content of silver chloride on the surface side on which the contact portion comes into contact with the living body is higher than the content of silver chloride on the inner side (the side closer to the interface with the substrate). Therefore, the contact with the living body is stabilized, and the vital sign information can be collected efficiently. Since the content of silver chloride is lower on the inner side, electron conduction is less inhibited by silver chloride, and the signal from the contact portion transfers through the inner side in which the content of silver is larger. Therefore, the signal of the vital sign information stably collected by the contact portion of the electrode layer can be efficiently outputted.

Preferably, in the contact portion of the electrode layer in the vital sign information measuring electrode according to the present invention, the content of silver on the inner side is higher than the content of silver on the surface side. When the content of silver on the inner side is higher than the content of silver on the surface side and the content of silver chloride on the surface side is high, the electric signal of the vital sign information is transferred by electron conduction efficiently on the inner side of the contact portion, and silver present on the inner side is unlikely to corrode. Therefore, the electrical conductivity of the contact portion is unlikely to deteriorate (change with time), and the service life of the vital sign information measuring electrode can be extended.

Preferably, in the vital sign information measuring electrode according to the present invention, the contact portion of the electrode layer has a concentration gradient region in which the content of silver chloride decreases gradually from the surface side toward the inner side and the content of silver increases gradually from the surface side toward the inner side. In the concentration gradient region, the concentration gradient of silver chloride in the thickness direction is opposite to the concentration gradient of silver in the thickness direction. When such a concentration gradient region is present, charge transfer by ions in which silver chloride is mainly involved is changed efficiently to charge transfer by electrons in which silver is mainly involved. Moreover, since there is no clear boundary between the region with a high silver chloride content on the surface side and the region with a high silver content on the inner side, delamination is unlikely to occur between these two region.

In the vital sign information measuring electrode according to the present invention, the substrate may be flexible. When the substrate is flexible, the vital sign information measuring electrode has high conformability to the measurement surface of a living body, and the contact portion can more easily conform to (come into contact with) the measurement surface. Therefore, the area of contact for the measurement increases. When the substrate is flexible, the vital sign information can be collected more efficiently and stably.

Preferably, in the contact portion of the electrode layer in the vital sign information measuring electrode according to the present invention, the silver or silver alloy is present as a silver or silver alloy powder dispersed in the synthetic resin, and the silver chloride in the contact portion is a chloride of the silver contained in the silver or silver alloy powder. When the silver or silver alloy contained in the electrode layer is in the form of a powder, electron conduction is stable in the powder. Therefore, when the powder dispersed in the synthetic resin has such a dispersion density that particles of the powder are in appropriate contact with each other in the synthetic resin, the signal can be transferred stably by electron conduction in the silver or silver alloy. Since the silver chloride is a chloride formed by chlorination of the silver contained in the powder, adhesion failure in the electrode layer is less likely to occur than when two layers, i.e., a layer containing a large amount of silver and a layer containing a large amount of silver chloride, are produced separately, and the layer obtained is robust against contact with a living body.

Preferably, in the vital sign information measuring electrode according to the present invention, the contact portion of the electrode layer has, on the surface side, a protective region that prevents corrosion of the silver on the inner side through the silver chloride. Silver easily corrodes, e.g., is easily oxidized or sulfurized. When the content of silver chloride in a region on the surface side of the contact portion is set to be sufficiently high, this region functions as a protective region, and corrosion of silver on the inner side of the contact portion can be prevented.

Another aspect of the present invention provides a method for producing a vital sign information measuring electrode including a substrate and an electrode layer disposed on a surface of the substrate, a part of the electrode layer serving as a contact portion that can come into contact with a living body. In the vital sign information measuring electrode, the contact portion of the electrode layer has a dispersion structure in which silver chloride and silver or a silver alloy are dispersed in a synthetic resin. In the contact portion of the electrode layer, the content of silver chloride on a surface side on which the contact portion comes into contact with the living body is higher than the content of silver chloride on an inner side. The method for producing the vital sign information measuring electrode includes: an application step of applying a resin-based composition containing a silver or silver alloy powder dispersed therein to the surface of the substrate to thereby obtain a coating; an electrically conductive layer forming step of curing the coating to form an electrically conductive layer adhering to the surface of the substrate; and a chlorination step of subjecting the electrically conductive layer to anodic electrolysis using a liquid containing chloride ions to convert part of the silver or silver alloy powder contained in the electrically conductive layer to silver chloride to thereby form the contact portion of the electrode layer from a first part of the electrically conductive layer.

The vital sign information measuring electrode production method includes: the application step of applying the resin-based composition containing the silver or silver alloy powder dispersed therein to the surface of the substrate; the electrically conductive layer forming step of curing the coating obtained in the application step to thereby form the electrically conductive layer; and the chlorination step of converting part of the silver or silver alloy powder contained in the electrically conductive layer to silver chloride. With this method, the electrode layer can be easily formed on the surface of the substrate, and the electrode layer having the dispersion structure in which silver chloride and silver or a silver alloy are dispersed in the synthetic resin can be easily formed. Moreover, by subjecting the silver contained in the silver or silver alloy powder to chlorination (anodic chlorination) through anodic electrolysis, the contact portion of the electrode layer in which the silver chloride content is higher on the surface side than on the inner side can be easily formed. Therefore, the vital sign information measuring electrode can be easily produced.

Preferably, in the chlorination step in the vital sign information measuring electrode production method according to the present invention, a concentration gradient region in which the content of silver chloride decreases gradually from the surface side toward the inner side and the content of silver increases gradually from the surface side toward the inner side is formed in the contact portion. In the anodic electrolysis in the chlorination step, the chlorination (anodic chlorination) proceeds from the surface side of the electrically conductive layer to the inner side, and therefore the concentration gradient region can be easily formed in the contact portion of the electrode layer subjected to the chlorination step.

Preferably, in the chlorination step in the vital sign information measuring electrode production method according to the present invention, a protective region that prevents corrosion of the silver on the inner side through the silver chloride is formed on the surface side of the contact portion. When the surface is subjected to anodic chlorination sufficiently through the anodic electrolysis in the chlorination step, the protective region can be easily formed on the surface side of the contact portion.

In the vital sign information measuring electrode production method according to the present invention, the substrate may have a flat plate shape, and the electrode layer may be disposed on a first principal surface of the substrate. Moreover, the electrode layer may include an external terminal for outputting an electric signal including vital sign information collected by the contact portion to the outside, and the external terminal may be formed from a second part of the electrically conductive layer, the second part being not subjected to the chlorination step. In this case, the first part of the electrically conductive layer formed in the electrically conductive layer forming step is subjected to the chlorination step to form the contact portion, and the second part of the electrically conductive layer is not subjected to the chlorination step and is used as the external terminal. Specifically, the electrically conductive layer serves as precursors of the contact portion and the external terminal. By forming the electrically conductive layer, the electrical contact between the contact portion and the external terminal in the electrode layer can be reliably established. Moreover, in the electrically conductive layer forming step, the electrically conductive layer can be formed on the first principal surface of the flat substrate. Therefore, the electrode layer including the contact portion and the external terminal can be produced reliably and efficiently.

Preferably, in the vital sign information measuring electrode production method according to the present invention, the electrode layer further includes a wiring portion that electrically connects the contact portion to the external terminal, and the wiring portion is formed from a third part of the electrically conductive layer formed in the electrically conductive layer forming step. In this case, the first part of the electrically conductive layer formed in the electrically conductive layer forming step is subjected to the chlorination step to form the contact portion, and the second part is not subjected to the chlorination step and serves as the external terminal. The wiring portion is formed from the third part other than the first and second parts. Specifically, the electrically conductive layer serves as precursors of the contact portion, the wiring portion, and the external terminal. By forming the electrically conductive layer, the electrical contact between the contact portion, the wiring portion, and the external terminal in the electrode layer can be reliably established. Since the electrically conductive layer is formed on the first principal surface of the flat substrate, the contact portion, the wiring portion, and the external terminal can be produced reliably and efficiently.

Preferably, in the chlorination step in the vital sign information measuring electrode production method according to the present invention, the third part of the electrically conductive layer is subjected to anodic electrolysis so that the wiring portion includes an electric conduction region based on the electrically conductive layer and a protective region formed on the surface side of the electric conduction region and containing the silver chloride. In the chlorination step, a portion of the electrically conductive layer formed in a region corresponding to the wiring portion is subjected to anodic electrolysis, and therefore the wiring portion of the electrode layer in which the silver chloride content is higher on the surface side than on the inner side can be easily formed. Moreover, the protective region containing silver chloride and formed on the surface side in the thickness direction of the wiring portion and the electric conduction region having a high silver content and located on the inner side can be easily formed.

### Advantageous Effects of Invention

The present invention provides a vital sign information measuring electrode that can measure vital sign information stably without using an electrically conductive gel. In the vital sign information measuring electrode provided in one preferred aspect, the silver or silver alloy contained in the electrode is unlikely to corrode, and therefore the measurement reliability of the vital sign information measuring electrode is unlikely to deteriorate even after repeated use. The present invention further provides a production method that can efficiently produce the vital sign information measuring electrode having the above-described structure.

### Brief Description of Drawings

[Fig. 1] Fig. 1(a) is a conceptual diagram illustrating the structure of a vital sign information measuring electrode according to an embodiment of the present invention, Fig. 1(b) is a cross-sectional view taken along line V1-V1 in Fig. 1(a), and Fig. 1(c) is a cross-sectional view taken along line V2-V2 in Fig. 1(a).
[Fig. 2] Fig. 2(a) is a conceptual diagram illustrating the flexibility of the vital sign information measuring electrode according to the embodiment of the present invention, Fig. 2(b) is a conceptual diagram illustrating an example of the use of the vital sign information measuring electrode according to the embodiment of the present invention, and Fig. 2(c) is a conceptual diagram illustrating the structure of an electrode unit including the vital sign information measuring electrode according to the embodiment of the present invention.
[Fig. 3] Fig. 3 is a flowchart of a method for producing the vital sign information measuring electrode 1 according to the embodiment of the present invention.
[Fig. 4] Fig. 4(a) is a cross-sectional view corresponding to the V2-V2 cross section of Fig. 1(a) and conceptually shows the state in which an application step in the method for producing the vital sign information measuring electrode 1 according to the embodiment of the present invention is performed, and Fig. 4(b) is a conceptual diagram illustrating the state in which an electrically conductive layer forming step in the production method according to the present embodiment has been completed.
[Fig. 5] Fig. 5(a) is a conceptual diagram illustrating a facility for a chlorination step, Fig. 5(b) is a conceptual diagram showing the state of a layered body 80 during the chlorination step in the production method according to the present embodiment, and Fig. 5(c) is an illustration in which the vital sign information measuring electrode 1 obtained through the chlorination step in the production method according to the present embodiment is placed so as to be easily compared with the layered body 80.
[Fig. 6] Fig. 6(a) is an observation image of a cross section of a vital sign information measuring electrode 1 in an Example, the cross section including the surface side of the vital sign information measuring electrode 1, Fig. 6(b) is a compositional image of silver (Ag) in the same viewing area as Fig. 6(a), and Fig. 6(c) is a compositional image of chlorine (Cl) in the same viewing area as Fig. 6(a).
[Fig. 7] Fig. 7(a) is a conceptual external view of the structure of a vital sign information measuring electrode 200 in Reference Example 1, and Fig. 7(b) is a conceptual external view of the structure of a vital sign information measuring electrode 300 in Reference Example 2.
[Fig. 8] Fig. 8(a) is a graph showing the results of measurement of changes in voltage when the vital sign information measuring electrodes in the Example, Comparative Example, and Reference Example were immersed in a physiological saline solution, and Fig. 8(b) is a graph showing the results of measurement of impedance when the vital sign information measuring electrodes in the Example, Comparative Example, and Reference Example were immersed in a physiological saline solution.
[Fig. 9] Fig. 9 is a table showing the results of a bending test on vital sign information measuring electrodes 1 in the Example.
[Fig. 10] Fig. 10 is a graph showing the results of measurement of brain waves using the vital sign information measuring electrode 1 in the Example and the vital sign information measuring electrode 200 in Reference Example 1.
[Fig. 11] Fig. 11(a) is an observation image of a cross section of the vital sign information measuring electrode 1 in the Example after a sulfurization test, and Fig. 11(b) is a compositional image of sulfur (S) in the same viewing area as Fig. 11(a).
[Fig. 12] Fig. 12(a) is an observation image of a cross section of a vital sign information measuring electrode in a Comparative Example after the sulfurization test, and Fig. 12(b) is a compositional image of sulfur (S) in the same viewing area as Fig. 12(a).
[Fig. 13] Fig. 13(a) is a table showing the results of measurement of the concentration of sulfur (S) on the surface side and inner side of cross sections of vital sign information measuring electrodes in the Example and Comparative Example after the sulfurization test, and Fig. 13(b) is a table showing the results of resistance measurement before and after the sulfurization test on vital sign information measuring electrodes in the Examples and Comparative Examples.
[Fig. 14] Fig. 14 is a graph showing the results of measurement of brain waves using the vital sign information measuring electrode 1 in the Example subjected to the sulfurization test and the vital sign information measuring electrode 300 in Reference Example 2.

### Description of Embodiments

Embodiments of the present invention will be described in detail with reference to the drawings.

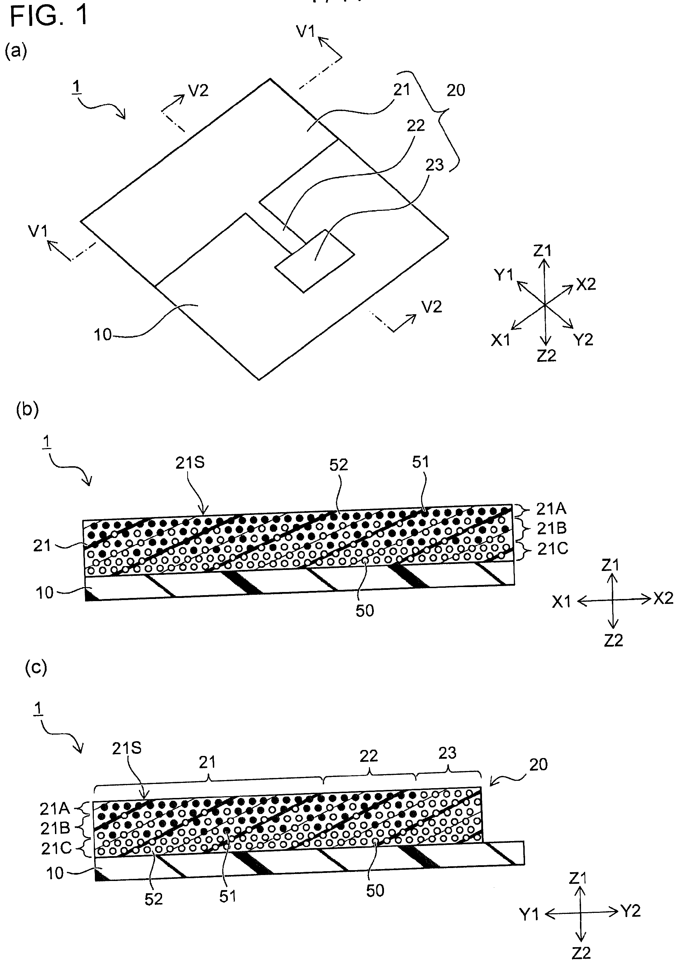

First, the structure of a vital sign information measuring electrode 1 according to an embodiment of the present invention will be described. Fig. 1(a) is a conceptual diagram illustrating the structure of the vital sign information measuring electrode 1 according to the embodiment of the present invention. Fig. 1(b) is a cross-sectional view taken along line V1-V1 in Fig. 1(a). Fig. 1(c) is a cross-sectional view taken along line V2-V2 in Fig. 1(a).

As shown in Fig. 1(a), the vital sign information measuring electrode 1 according to the embodiment of the present invention includes a substrate 10 and an electrode layer 20 disposed on a surface of the substrate 10.

In Fig. 1, the substrate 10 of the vital sign information measuring electrode 1 is composed of a cloth having a flat plate shape and having a surface coated with polyurethane. In the embodiment of the present invention, since the substrate 10 is made of a cloth, the vital sign information measuring electrode 1 as a whole is flexible. The substrate 10 may have any specific shape and may be formed of any material. The shape of the substrate 10 is not limited to a flat plate shape and may be, for example, a rod-like shape, a lump shape, or a complicated shape including a base portion and a plurality of leg portions extending from the base portion (for example, the shape of a vital sign information measuring electrode 200 according to a Reference Example described later). For example, the material forming the substrate 10 may be an electrically insulating material such as the cloth described above or may be an electrically conductive material such as aluminum or an electrically conductive carbon material.

As shown in Fig. 1(a), the electrode layer 20 of the vital sign information measuring electrode 1 has a contact portion 21 that can be in contact with a living body, a wiring portion 22 having a first end connected to the contact portion 21, and an external terminal 23 connected to a second end of the wiring portion 22.

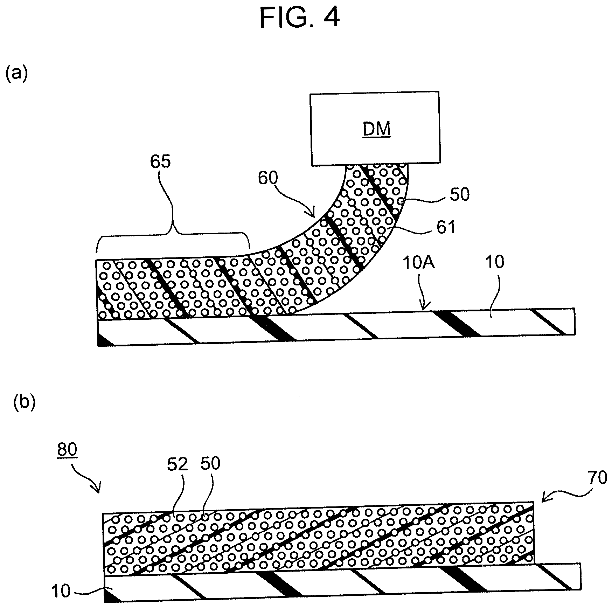

The wiring portion 22 of the electrode layer 20 electrically connects the contact portion 21 to the external terminal 23. As shown in Fig. 1(b), the contact portion 21 of the electrode layer 20 has a dispersion structure in which a silver or silver alloy powder (hereinafter referred to as a silver powder 50) represented by open circles " 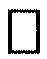 " in Fig. 1(b) and a silver chloride (AgCl) powder (hereinafter referred to as a silver chloride powder 51) represented by solid circles " 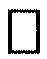 " in Fig. 1(b) are dispersed in a synthetic resin 52. The silver powder 50 is composed of silver or a silver alloy and may be a powder composed only of silver or a silver alloy powder containing copper, palladium, etc. A mixture of a silver powder and a silver alloy powder may be used. In Fig. 1(b), for ease of understanding of the illustration, the thickness (the length in a Z1-Z2 direction) of the electrode layer 20 is larger than the thickness of the substrate 10, but this is not a limitation. Rather, the thickness of the substrate 10 may be several hundreds of micrometers, and the thickness of the electrode layer 20 may be several tens of micrometers. In this case, they can often be handled with ease. This also applies to Fig. 1(c) and cross-sectional views in Fig. 4 and subsequent figures.

The distribution of silver and the distribution of silver chloride in the contact portion 21 are nonuniform in the thickness direction. The contact portion 21 has a surface 21S that is to be in contact with a living body, and the content of silver chloride on the surface 21S side is higher than the content of silver chloride on the inner side (the side closer to the substrate 10). Specifically, as shown in Figs. 1(b) and 1(c), the contact portion 21 has, on the surface 21S side, a region (silver chloride region) 21A in which the density of the silver chloride powder 51 present is higher than other regions. The content of silver is higher on the inner side than on the surface 21S side. Specifically, as shown in Figs. 1(b) and 1(c), the contact portion 21 has, on the inner side, a region (silver region) 21C in which the density of the silver powder 50 present is higher than other regions. The contact portion 21 has, between the silver chloride region 21A and the silver region 21C, a concentration gradient region 21B in which the content of silver chloride decreases gradually from the surface 21S side to the inner side and the content of silver increases gradually from the surface 21S side to the inner side.

The vital sign information measuring electrode 1 is a device that has the contact portion 21 to be in contact with a living body to collect vital sign information such as pulse waves, cardioelectric potential, myoelectric potential, body mass index, or brain waves as an electric signal and outputs the electric signal from the external terminal 23 to the outside. In the electrode layer 20 of the vital sign information measuring electrode 1, charge transfer on the surface 21S in contact with the living body is effected by ions (chloride ions). Therefore, on the surface 21S of the contact portion 21, a metal chloride that can easily release chloride ions is suitable and is an essential material. In particular, silver chloride is preferable because it can be easily produced and can be handled with ease. In the contact portion 21 of the electrode layer 20 of the vital sign information measuring electrode 1, the content of silver chloride (the density of the silver chloride powder 51 present) on the side toward the surface 21S that is to be in contact with the living body is higher than the content of silver chloride (the density of the silver chloride powder 51 present) on the inner side. Therefore, the contact with the living body is stabilized, and the vital sign information can be collected efficiently.

In the contact portion 21, since the content of silver chloride (the density of the silver chloride powder 51 present) is lower on the inner side, electron conduction is less inhibited by silver chloride. In this case, in the contact portion 21, the electric signal of the vital sign information collected at the surface 21S transfers through the inner side with a high silver content (a high density of the silver powder 50 present). Therefore, in the vital sign information measuring electrode 1, the signal of the vital sign information can be stably and efficiently collected and outputted by simply bringing the contact portion 21 into direct contact with the living body without using an electrically conductive gel. This will be described in more detail in EXAMPLES.

In the contact portion 21, the content of silver (the density of the silver powder 50 present) on the inner side is higher than the content of silver (the density of the silver powder 50 present) on the surface 21S side, and the content of silver chloride (the density of the silver chloride powder 51 present) on the surface 21S side is higher. On the inner side of the contact portion 21, the electric signal of the vital sign information is efficiently transferred by electron conduction, and silver present inside the contact portion 21 is unlikely to corrode. As described above, in the contact portion 21 of the vital sign information measuring electrode 1, a region including the silver region 21C on the inner side functions as an electric conduction region that conveys the signal to the external terminal 23, and a region including the silver chloride region 21A on the surface 21S side functions not only as a region for collecting the vital sign information but also as a protective region for preventing corrosion of silver in the electric conduction region. Therefore, the electric conductivity of the contact portion 21 is unlikely to deteriorate (change with time), and the service life of the vital sign information measuring electrode 1 can be extended.

As described above, the contact portion 21 has the concentration gradient region 21B in which the distribution of the content of silver chloride (the density of the silver chloride powder 51 present) in the thickness direction differs from the distribution of the content of silver (the density of the silver powder 50 present) in the thickness direction. Therefore, charge transfer by ions in which silver chloride is mainly involved is changed efficiently to charge transfer by electrons in which silver is mainly involved.

In the contact portion 21, since there is no clear boundary between the region with a high silver chloride content on the surface 21S side (the silver chloride region 21A) and the region with a high silver content on the inner side (the silver region 21C), delamination is less likely to occur between these two region than when two layer are stacked to form the contact portion 21.

The silver or silver alloy in the contact portion 21 of the electrode layer 20 is present as the silver powder 50 dispersed in the synthetic resin 52, and the silver chloride in the contact portion 21 is a chloride formed by chlorination of the silver contained in the silver powder 50. Specifically, the silver chloride powder 51 contained in the contact portion 21 of the electrode layer 20 is derived from the silver contained in the silver powder 50 dispersed in the synthetic resin 52 and is composed of a chloride formed through a chemical reaction of the silver. In this case, since the silver chloride powder 51 is formed by diffusion of chloride ions inside a member containing the synthetic resin 52 and the silver powder 50 dispersed therein (the entire portion of this member has the structure of the silver region 21C), no clear boundary is formed between the silver region 21C and the silver chloride region 21A, and the concentration gradient region 21B is formed inevitably. Therefore, in contrast to the case where the electrode layer 20 is composed of two separately formed layers including a layer containing a large amount of silver and a layer containing a large amount of silver chloride, a problem such as delamination does not occur in the electrode layer 20, and therefore the electrode layer 20 obtained is robust against contact with a living body.

The contact portion 21 including the silver chloride region 21A and the silver region 21C can be formed by causing the member containing the synthetic resin 52 and the silver powder 50 dispersed therein to be exposed to a chloride ion-containing environment (specifically, for example, the member is subjected to anodic oxidation using a liquid containing chloride ions described later). In this case, the contact portion 21 of the electrode layer 20 can be formed more easily than when a layer corresponding to the silver chloride region 21A and a layer corresponding to the silver region 21C are produced separately and then stacked. When the contact portion 21 is formed by the above method, the diffusion of chloride ions proceeds from the surface side. Therefore, the structure having the silver chloride region 21A on the surface 21S side and the silver region 21C on the inner side can be easily obtained.

The contact portion 21 of the electrode layer 20 that includes the silver chloride region 21A on the surface 21S side is advantageous also in terms of corrosion protection. Specifically, silver easily corrodes, e.g., is easily oxidized or sulfurized. When oxide or sulfide is formed, it impedes electron conduction through silver. It is therefore preferable that silver located in the silver region 21C of the electrode layer 20 is not corroded. The silver contained in the silver chloride powder 51 has already been chlorinated, and therefore oxidation and sulfurization of silver are unlikely to occur in the silver chloride region 21A with a high density of the silver chloride powder 51 present. Specifically, the silver chloride region 21A located on the surface 21S side of the electrode layer 20 functions as a protective region that prevents corrosion of silver present on the inner side of the electrode layer 20. Therefore, when the silver chloride region 21A is located on the surface 21S side of the electrode layer 20, not only the electric signal can be easily received from the living body, but also corrosion of the electrode layer 20 is prevented, so that the durability of the vital sign information measuring electrode 1 can be improved.

As shown in Fig. 1(c), the wiring portion 22 of the electrode layer 20 has the same structure as the contact portion 21. Specifically, the wiring portion 22 has the dispersion structure in which the silver powder 50 and the silver chloride powder 51 are dispersed in the synthetic resin 52. A region with a high silver chloride content is disposed on the surface side, and a region with a high silver content is disposed on the inner side. A concentration gradient region in which the distribution of the silver chloride content in the thickness direction differs from the distribution of the silver content in the thickness direction is present between these regions. Therefore, the wiring portion 22 can be produced by the same method as the method for producing the contact portion 21. In the wiring portion 22 also, the silver chloride region 21A prevents corrosion of silver present on the inner side.

Finally, as shown in Fig. 1(c), the external terminal 23 has a dispersion structure in which the silver powder 50 is dispersed in the synthetic resin 52, i.e., the same structure as the structure of the silver region 21C. When the contact portion 21, the wiring portion 22, and the external terminal 23 have the structures described above, the contact portion 21 and the wiring portion 22 can be formed differently from the external terminal 23 depending on whether or not chlorination treatment of silver is performed, as described later.

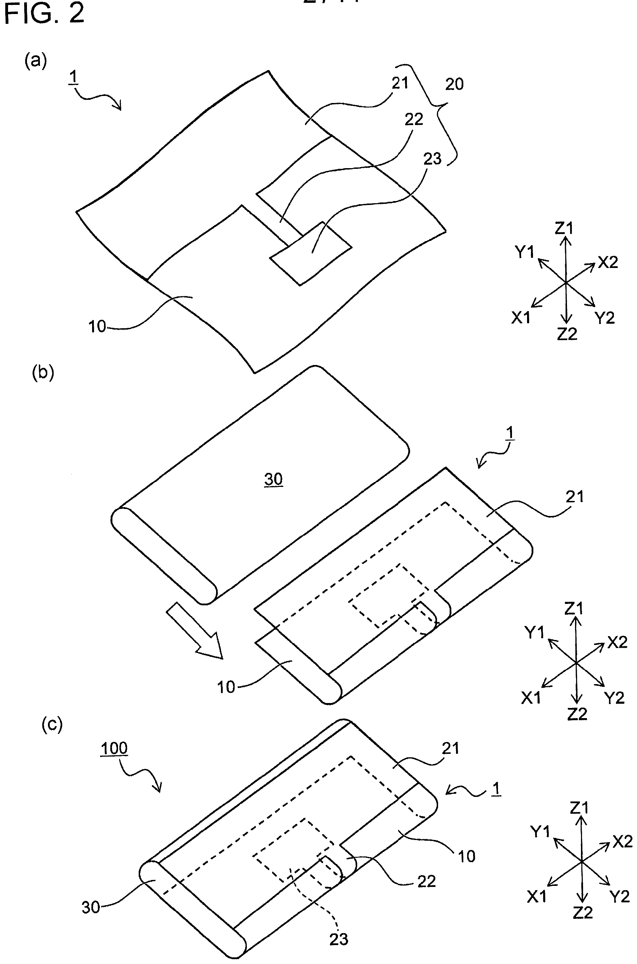

Next, the (above-described) fact that the entire vital sign information measuring electrode 1 is flexible will be further described using Fig. 2. Fig. 2(a) is a conceptual diagram illustrating the flexibility of the vital sign information measuring electrode 1 according to the embodiment of the present invention. Fig. 2(b) is a conceptual diagram illustrating an example of the use of the vital sign information measuring electrode 1 according to the embodiment of the present invention. Fig. 2(c) is a conceptual diagram illustrating the structure of an electrode unit 100 including the vital sign information measuring electrode 1 according to the embodiment of the present invention.

As described above, the substrate 10 is formed of a cloth and is flexible. The electrode layer 20 has the structure in which the silver powder 50 and the silver chloride powder 51 are dispersed in the matrix formed of the synthetic resin 52, so the electrode layer 20 is also flexible. In particular, a polyester resin, a rubber-modified urethane resin, etc. is preferably used as the synthetic resin 52. Therefore, as shown in Fig. 2(a), the vital sign information measuring electrode 1 as a whole is flexible. In this case, the contact portion 21 of the vital sign information measuring electrode 1 has high conformability to the measurement surface of a living body and can more easily conform to (come in contact with) the measurement surface. Since the substrate 10 is flexible as described above, the measurement contact area of the vital sign information measuring electrode 1 is large, and the vital sign information can be collected more efficiently and stably.

Since the vital sign information measuring electrode 1 is flexible, it can be bent for use as shown in Fig. 2(b). The contact portion 21 and the external terminal 23 are formed on one side of the flat substrate 10. However, even in this case, by bending the vital sign information measuring electrode 1 so as to be folded, the contact portion 21 and the external terminal 23 can be opposed to each other. The contact portion 21 faces a living body. Therefore, in the above state, the external terminal 23 is distal from the living body and can therefore be handled with ease. From the viewpoint of stabilizing the bent state, a spacer 30 formed of, for example, an elastic body may be placed so as to be in contact with the inner surface side of the vital sign information measuring electrode 1. Although the thus-obtained electrode unit 100 (see Fig. 2(c)) has a simple structure, its handleability is high.

Next, a method for producing the vital sign information measuring electrode 1 will be described. No limitation is imposed on the method for producing the vital sign information measuring electrode 1. However, when a method described below is used for the production, the vital sign information measuring electrode 1 can be produced efficiently.

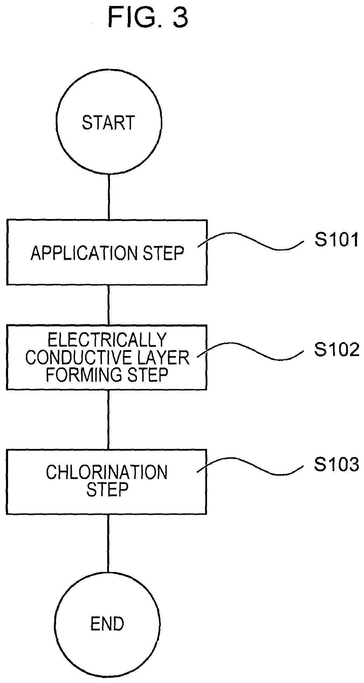

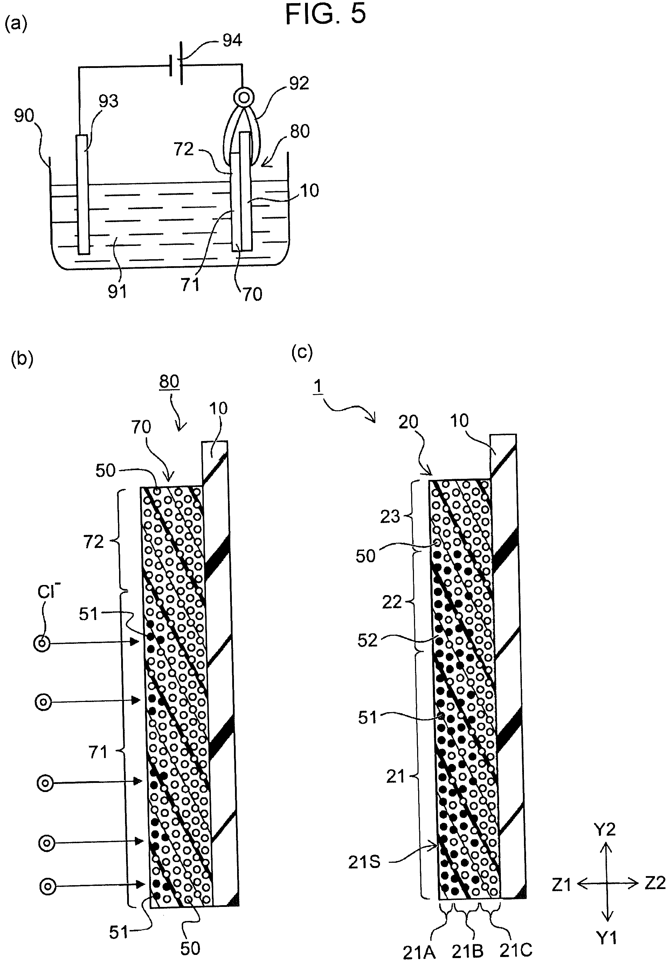

Fig. 3 is a flowchart of the method for producing the vital sign information measuring electrode 1 according to the embodiment of the present invention. Fig. 4(a) is a conceptual cross-sectional view (illustration) taken along V2-V2 in Fig. 1(a) (the same applies to cross-sectional views in Fig. 4(b) and subsequent figures), showing an application step in the method for producing the vital sign information measuring electrode 1 according to the embodiment of the present invention. Fig. 4(b) is a conceptual diagram showing the state in which an electrically conductive layer forming step in the production method according to the present embodiment has been completed. Fig. 5(a) is a conceptual diagram showing a facility for a chlorination step. Fig. 5(b) is a conceptual diagram showing the state of a layered body 80 during the chlorination step in the production method according to the present embodiment. Fig. 5(c) is an illustration in which the vital sign information measuring electrode 1 obtained through the chlorination step in the production method according to the present embodiment is placed so as to be easily compared with the layered body 80.

As shown in Fig. 3, the method for producing the vital sign information measuring electrode 1 according to the embodiment of the present invention includes the application step (step S101), the electrically conductive layer forming step (step S102), and the chlorination step (step S103).

First, in the application step, a resin-based composition 60 in which the silver powder 50 is dispersed in an uncured resin composition 61 is applied to a surface of the substrate 10 using an application device DM, as shown in Fig. 4(a). A coating 65 on the substrate 10 is thereby obtained. Specific examples of the application device DM include a screen printer, an inkjet device, and a dispenser.

The uncured resin composition 61 is a material capable of forming the synthetic resin 52 after curing and is softer than the synthetic resin 52 when the uncured resin composition 61 is heated to its softening temperature or higher in the application step or contains a non-polymerized material, a solvent, etc. Therefore, the resin-based composition 60 obtained by dispersing the silver powder 50 in the uncured resin composition 61 can be formed into the coating 65 having any shape and any thickness using the application device DM. In the illustration in Fig. 4(a), the coating 65 of the resin-based composition 60 supplied to a first principal surface 10A of the substrate 10 is formed in part of a region in which the contact portion 21 is to be eventually formed, and the resin-based composition 60 is to be successively supplied to other regions from the application device DM.

When the resin-based composition 60 contains a solvent, no limitation is imposed on the type of solvent. When the solvent can dissolve a material located on a surface of the substrate 10 (specifically, the first principal surface 10A of the substrate 10) (e.g., an overcoat layer of the cloth), the adhesion of the coating 65 formed on the substrate 10, an electrically conductive layer 70 (see Fig. 4(b)) formed from the coating 65, and the electrode layer 20 (see Fig. 5(c)) formed from the electrically conductive layer 70 to the substrate 10 can be increased. Therefore, even when the substrate 10 is flexible and is bent, the electrode layer 20 is unlikely to be delaminated from the substrate 10, and the vital sign information measuring electrode 1 as a whole can have high flexibility.

Next, in the electrically conductive layer forming step, the coating 65 formed on the substrate 10 is cured to form the electrically conductive layer 70 adhering to the surface of the substrate 10 (specifically, the first principal surface 10A of the substrate 10). The electrically conductive layer 70 has a structure in which the silver powder 50 is dispersed in the synthetic resin 52. The method for curing the coating 65 is appropriately set depending on the uncured resin composition 61. Specifically, the synthetic resin 52 may be obtained by cooling the uncured resin composition 61 to lower than its softening temperature or may be obtained by volatilizing the solvent contained in the uncured resin composition 61. Alternatively, the synthetic resin 52 may be obtained by polymerizing an unpolymerized material contained in the uncured resin composition 61. The synthetic resin 52 may be obtained using a combination of these methods (cooling, volatilization, and polymerization). In this manner, the layered body 80 is obtained. In the layered body 80, the electrically conductive layer 70 having a shape and a thickness corresponding to those of the electrode layer 20 and having a uniform composition that is the same as the composition of the silver region 21C is formed on the first principal surface 10A of the substrate 10.

Finally, in the chlorination step, part of the electrically conductive layer 70 is subjected to anodic electrolysis. Specifically, as shown in Fig. 5(a), a solution containing chloride ions (this solution is hereinafter referred to as an electrolyte 91) is placed in a treatment bath 90, and the layered body 80 held by an electrically conductive clip 92 and a cathode 93 are immersed in the electrolyte 91 and connected to a power source 94 through wiring lines. No particular limitation is imposed on the composition of the electrolyte 91 so long as it contains chloride ions. Examples of the counter cations include sodium ions and potassium ions. The content of the chloride ions is set appropriately. A nonlimiting example of the content is about 1M. The solvent for the electrolyte 91 may by water, and the pH of the solvent may be neutral. Any material having electrical conductivity may be used as the material forming the cathode 93, and examples include a carbon electrode.

As shown in Fig. 5(a), the layered body 80 is partially but not completely immersed in the electrolyte 91. Specifically, a portion corresponding to the contact portion 21 and the wiring portion 22 (this portion is hereinafter referred to as a first portion 71) is immersed in the electrolyte 91, but a portion corresponding to the external terminal 23 (this portion is hereinafter referred to as a second portion 72) is not immersed in the electrolyte 91. If necessary, the second portion 72 may be masked in order to prevent splashes caused by bubbles generated by electrolysis from coming into contact with the second portion 72.

The anodic electrolysis is performed with the electrically conductive layer 70 of the layered body 80 partially immersed in the electrolyte 91. Then, as shown in Fig. 5(b), chloride ions (Cl⁻) reach the first portion 71 and diffuse from the surface side of the first portion 71 to the inner side. Therefore, the chlorination reaction of silver contained in the silver powder 50 proceeds from the surface side to the inner side, and the silver chloride powder 51 is formed from the silver powder 50 successively from the surface side to the inner side. In this case, as shown in Fig. 5(c), in a portion corresponding to the first portion 71, the silver chloride region 21A is formed on the surface 21S side, and the concentration gradient region 21B is formed on the inner side. By appropriately setting the conditions for the anodic electrolysis, a region in which substantially no chlorination reaction has proceeded can remain present on the inner side of the electrically conductive layer 70, and this region serves as the silver region 21C. The contact portion 21 is thereby formed, and the wiring portion 22 having the same structure as the contact portion 21 is formed. The second portion 72 not subjected to the anodic electrolysis serves as the external terminal 23. The vital sign information measuring electrode 1 with the electrode layer 20 on the surface of the substrate 10 is thereby obtained. In the chlorination step, a region having the same structure as the silver chloride region 21A of the contact portion 21 is formed in the wiring portion 22 of the electrode layer 20. This region is provided as a protective region that exerts the corrosion protection function instead of the function of collecting the vital sign information, which is the basis function of the silver chloride region 21A of the contact portion 21.

The conditions for the anodic electrolysis (such as the temperature of the electrolyte 91, current density, and total current amount) are set appropriately in consideration of the composition and thickness of the electrically conductive layer 70 and the compositions and thicknesses of the silver chloride region 21A, the concentration gradient region 21B, and the silver region 21C formed in the chlorination step. If the current density is excessively low, the processing time is long. If the current density is excessively high, the possibility that the uniformity of the processing may deteriorate (so-called "burnt **phenomenon"** may be formed) increases. In consideration of the above facts, the current density is, for example, preferably from about 10 mA/cm² to about 250 mA/cm² in some cases.

As described above, with the production method according to the present embodiment, the electrode layer 20 can be easily formed on the surface of the substrate 10 (specifically the first principal surface 10A of the substrate 10), and the electrode layer 20 having the dispersion structure in which the silver powder 50 and the silver chloride powder 51 are dispersed in the synthetic resin 52 can be easily formed. Moreover, by chlorinating (anodically chlorinating) silver included in the silver powder 50 by anodic electrolysis, the contact portion 21 of the electrode layer 20 in which the content of silver chloride is higher on the surface 21S side than on the inner side can be formed easily. Since chloride ions diffuse from the surface side of the first portion 71 of the electrically conductive layer 70 during the anodic electrolysis to thereby form silver chloride, the contact portion 21 having the concentration gradient region 21B can be easily formed. The thickness of the concentration gradient region 21B and the magnitude of the concentration gradient can be easily controlled with high reproducibility by appropriately setting the electrolysis conditions. Therefore, the contact portion 21 having the silver region 21C on the inner side of the concentration gradient region 21B can be easily formed. With the production method according to the present embodiment, the contact portion 21 in which the silver content on the inner side is higher than the silver content on the surface side can be easily formed. As described above, with the production method according to the present embodiment, the vital sign information measuring electrode 1 can be easily produced.

The embodiments described above are described for facilitating an understanding of the present invention and do not limit the present invention. Therefore, the elements disclosed in the above embodiments are intended to encompass all design modifications and equivalents belonging to the technical scope of the present invention.

### EXAMPLES

The present invention will next be described more specifically by way of Examples etc., but the scope of the present invention is not limited to the Examples.

First, the vital sign information measuring electrode 1 was produced, and the produced vital sign information measuring electrode was observed.

To produce the vital sign information measuring electrode 1, a substrate 10 formed of a cloth (thickness: 500 **µ**m) coated with polyurethane was prepared. A polymer-type electrically conductive paste containing the silver powder 50 (the resin-based composition 60) was applied (by screen printing) to the first principal surface 10A of the substrate 10 to thereby form a coating 65 having a shape corresponding to the shape of the electrode layer 20. The product was treated under prescribed conditions (at 130°C for 30 minutes) to cure the coating 65, and an electrically conductive layer 70 having a thickness of 10 **µ**m to 15 **µ**m was thereby formed. A layered body 80 including the substrate 10 and the electrically conductive layer 70 disposed on the first principal surface 10A of the substrate 10 was thereby obtained.

The device shown in Fig. 5(a) was used to immerse the layered body 80 in an electrolyte (a 1M aqueous sodium chloride solution) 91 containing chloride ions (Cl⁻). In this case, the electrically conductive layer 70 was immersed such that only the first portion 71 corresponding to the contact portion 21 and the wiring portion 22 was immersed. Then the first portion 71 of the electrically conductive layer 70 was subjected to anodic electrolysis with the current density set within the range of 50 mA/cm² to 200 mA/cm². In the electrically conductive layer 70 originally having white color, the first portion 71 subjected to anodic electrolysis was turned black.

A vital sign information measuring electrode 1 was thereby obtained in which the electrode layer 20 including the contact portion 21 and the wiring portion 22 formed from the first portion 71 and the external terminal 23 formed from the second portion 72 was disposed on the first principal surface 10A of the substrate 10.

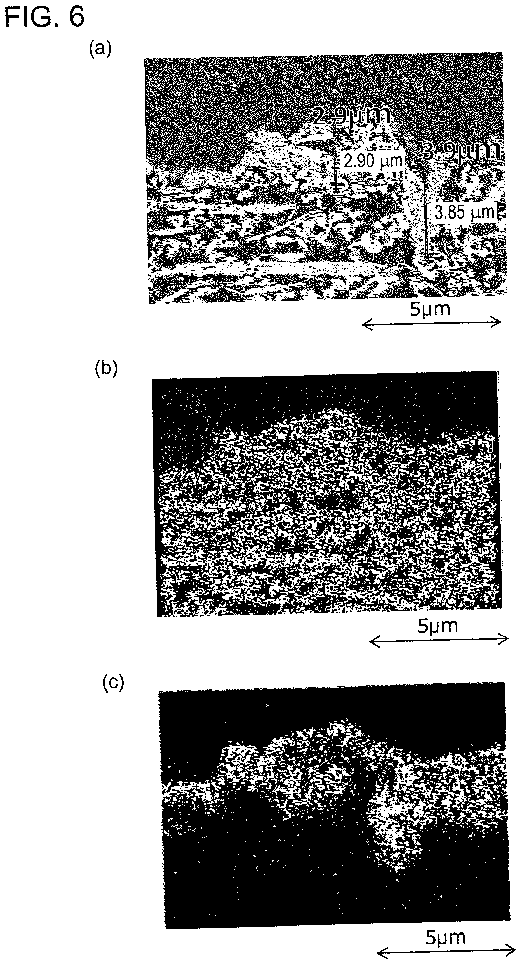

A cross section of a portion including the surface 21S of the contact portion 21 of the electrode layer 20 of the vital sign information measuring electrode 1 obtained was subjected to observation and compositional analysis using a scanning electron microscope and an analyzer included in the scanning electron microscope. The results are shown in Fig. 6. Fig. 6(a) is an observation image of the cross section of the portion including the surface 21S of the contact portion 21 of the electrode layer 20. Fig. 6(b) is a compositional image of silver (Ag) (white regions in the figure are silver) in the same viewing area as Fig. 6(a), and Fig. 6(c) is a compositional image of chlorine (Cl) (white regions in the figure are chlorine) in the same viewing area as Fig. 6(a).

As shown in Fig. 6(a), it was found that fine powdery aggregates with outer shapes different from the shape of the silver powder 50 were formed to a depth of about 3 **µ**m to about 4 **µ**m from the surface 21S. As shown in Fig. 6(b), it was found that silver (Ag) was present over the entire electrode layer 20 in the viewing area although the concentration distribution was not uniform. As shown in Fig. 6(c), it was found that chlorine (Cl) was present in positions corresponding to the aggregates found in Fig. 6(a). It was therefore found that the aggregates found in Fig. 6(a) were silver chloride. Specifically, these aggregates are the silver chloride powder 51.

As can be seen from the distribution of chlorine shown in Fig. 6(c), silver chloride is present so as to cover the surface 21S of the contact portion 21 of the electrode layer 20 and is present at a high concentration in a region extending inward and having a thickness of about 0.5 **µ**m. However, almost no chlorine (Cl) is found in an inner region deeper than a position at about 5 **µ**m from the surface 21S of the contact portion 21 of the electrode layer 20. In a region between these regions (a region with a depth of from 0.5 **µ**m to 5 **µ**m from the surface 21S), there are a plurality of areas in which chlorine (Cl) penetrates to a depth of several tens of micrometers. On average, the density of chlorine (Cl) gradually decreases from the surface 21S side toward the inner side, and the density of silver (Ag) increases gradually from the surface 21S side toward the inner side. In the electrode layer 20 of the contact portion 21 of the vital sign information measuring electrode 1 used for the measurement, a region extending from the surface 21S and having a thickness of about 0.5 **µ**m was found to be the silver chloride region 21A. A region extending from the inner side of the silver chloride region 21A to a depth of 5 **µ**m from the surface 21S of the contact portion 21 was found to be the concentration gradient region 21B, and a region on the inner side of the concentration gradient region 21B was found to be the silver region 21C.

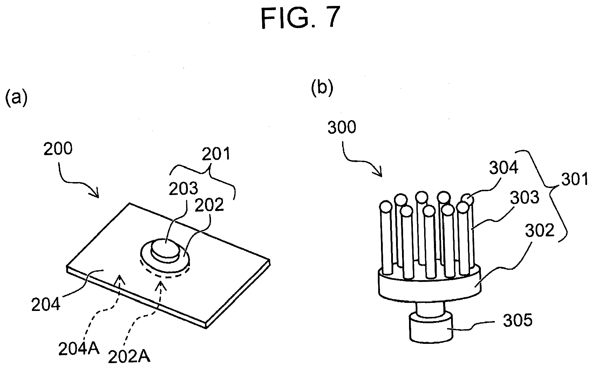

Next, the vital sign information measuring electrode 1 obtained was used for measurement. For the purpose of comparison, a layered body 80 not subjected to the chlorination step (Comparative Example), a commercial vital sign information measuring electrode 200 having a structure shown in Fig. 7(a) (Reference Example 1), and a vital sign information measuring electrode 300 having a structure shown in Fig. 7(b) according to another Reference Example (Reference Example 2) were used.

The entire contact portion of the electrode layer of the vital sign information measuring electrode according to the Comparative Example has a structure in which the silver powder 50 is dispersed in the synthetic resin 52. The vital sign information measuring electrode 200 according to Reference Example 1 has a structure in which an electrode portion 201 formed of an electrically conductive carbon material passes through an adhesive sheet 204 in its thickness direction and is fixed thereto. The electrode portion 201 includes: a contact portion 202 that is entirely embedded in the adhesive sheet 204 and having a first surface 202A exposed at an adhesive surface 204A of the adhesive sheet 204; and an external terminal 203 that is disposed on a surface of the contact portion 202 opposite to the first surface 202A and is electrically connected to the contact portion 202. During use, the first surface 202A of the contact portion 202 is a surface in contact with a living body. Therefore, before use, an electrically conductive gel is applied so as to cover the entire first surface 202A of the contact portion 202. The vital sign information measuring electrode 300 according to Reference Example 2 includes a metallic electrode portion 301 and a terminal portion 305 electrically connected to the electrode portion 301. The electrode portion 301 includes: a disk-shaped base portion 302; nine pins 303 extending from an end surface of the base portion 302 that is opposite to an end surface on which the terminal portion 305 is disposed; and spherical bodies 304 disposed at forward ends of the respective pins 303. The spherical bodies 304 are portions to be in contact with a living body. From the viewpoint of preventing corrosion and obtaining electrical continuity between the vital sign information measuring electrode 300 and the living body, the entire outermost surfaces of the spherical bodies 304 are plated with gold.

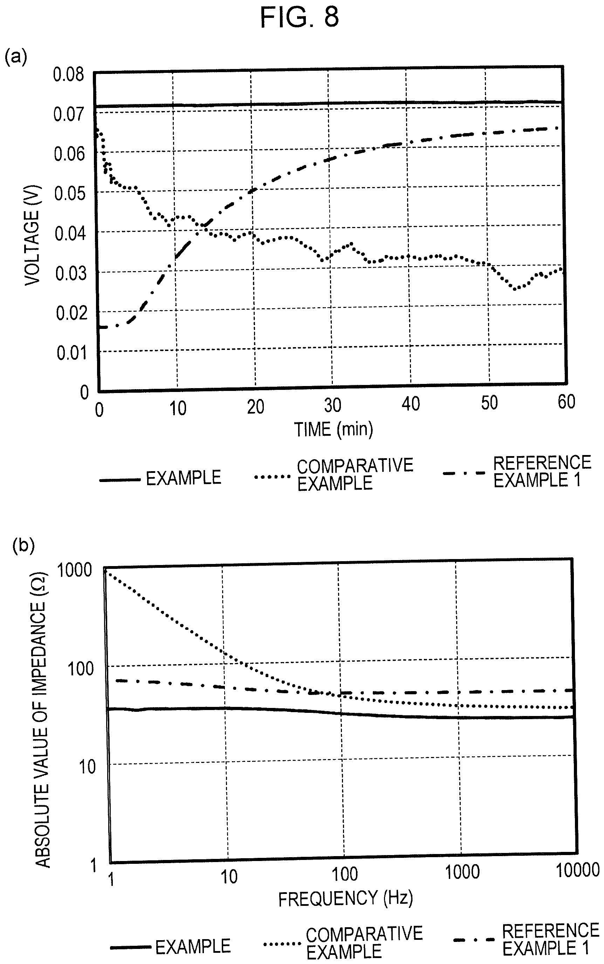

First, the vital sign information measuring electrode 1 obtained was immersed in a saline solution (a 0.9% aqueous sodium chloride solution), and changes in the voltage of the vital sign information measuring electrode 1 over time were measured (reference electrode: silver-silver chloride standard electrode). The results of the measurement are shown in Fig. 8(a). As shown in Fig. 8(a), the voltage of the vital sign information measuring electrode 1 according to the Example was about 0.07 V and was approximately constant over the entire measurement period (60 minutes).

Changes in the voltage of each of the vital sign information measuring electrode according to the Comparative Example and the vital sign information measuring electrode 200 according to Reference Example 1 in a saline solution over time were measured in the same manner as for the vital sign information measuring electrode 1 according to the Example. As shown in Fig. 8(a), in the vital sign information measuring electrode according to the Comparative Example, the voltage decreased with time, and the voltage drop in a measurement period of 60 minutes was found to be 0.03 V or more. In the vital sign information measuring electrode 200 according to Reference Example 1, the voltage increased with time, and the voltage rise in a measurement period of 60 minutes was found to be about 0.045 V. These results show that, in the vital sign information measuring electrode 1 according to the Example, surface polarization is unlikely to occur when the electrode 1 is in contact with an electrolyte such as a saline solution and the measurement stability of the electrode 1 is high.

Next, the impedance of each of the vital sign information measuring electrode 1 according to the Example, the vital sign information measuring electrode according to the Comparative Example, and the vital sign information measuring electrode 200 according to Reference Example 1 was measured in a saline solution. The frequency characteristics of the measurement results are shown in Fig. 8(b). As shown in Fig. 8(b), in the vital sign information measuring electrode according to the Comparative Example, the absolute value of the impedance tends to increase as the frequency decreases, and the resistance component was found to be large. In each of the vital sign information measuring electrode 1 according to the Example and the vital sign information measuring electrode 200 according to Reference Example 1, no noticeable frequency dependence of the absolute value of the impedance was found, and the resistance component was found to be small. These results show that, unlike the vital sign information measuring electrode according to the Comparative Example, the resistance of the vital sign information measuring electrode 1 according to the Example at the interface with the electrolyte is small and is comparable to the resistance of the vital sign information measuring electrode 200 according to Reference Example 1 at the interface between the surface of **gold-plated** and the electrolyte.

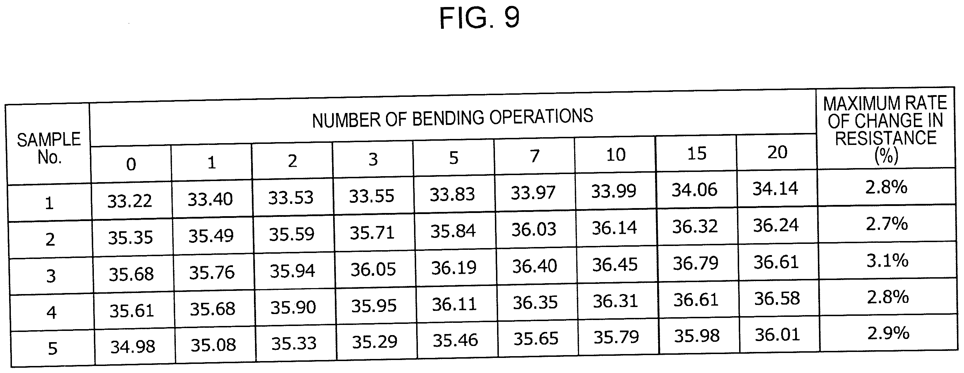

Next, the flexibility of the vital sign information measuring electrode 1 according to the Example was examined (the number of samples: 5). The vital sign information measuring electrode 1 was folded (bent 180 degrees) around a pin with a diameter of 1 mm, and this state was held for 60 seconds to perform the bending test at a radius of curvature of 0.5 mm. After the bending test, the vital sign information measuring electrode 1 was unfolded on a flat substrate, and the surface resistivity (unit: **Ω**/square) across the bent portion was measured. The bending test was repeated, and the surface resistivity after the bending test was measured similarly. The bending test and the measurement of the surface resistivity were repeated 15 times. The results of the test and the results of the measurement of the surface resistivity before the bending test are shown in Fig. 9. As shown in Fig. 9, the maximum value of the rate of change in surface resistivity ([the surface resistivity before the bending test / the surface resistivity after the bending test - 1] × 100, unit: %) (the maximum rate of change in resistance, unit: %) was about 3%. It was found that the resistivity of the vital sign information measuring electrode 1 according to the Example is almost unchanged even after bending, i.e., the vital sign information measuring electrode 1 is excellent in flexibility.

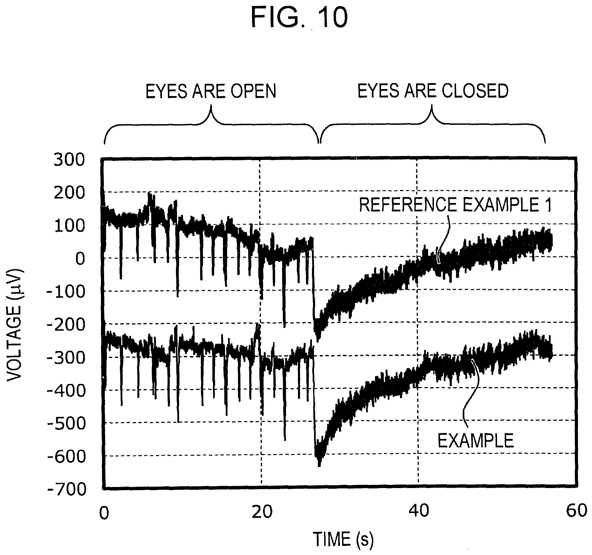

Next, the vital sign information measuring electrode 1 according to the Example was used to form the electrode unit 100 shown in Fig. 2(c), and the electrode unit 100 was fixed to the forehead of a human. Then vital sign information was measured with an earlobe grounded. For the purpose of comparison, vital sign information was measured similarly using the vital sign information measuring electrode 200 according to Reference Example 1 instead of the electrode unit 100. Before the measurement using the vital sign information measuring electrode 200 in Reference Example 1, an electrically conductive gel was applied between the vital sign information measuring electrode 200 and the forehead of the human. The results of the measurement are shown in Fig. 10.

As shown in Fig. 10, in the results of the measurement using the vital sign information measuring electrode 200 according to Reference Example 1, changes in voltage with the eyes open (when the eyes are open) differ from changes in voltage with the eyes closed (when the eyes are closed), so that the measurement of the brain waves allows identification as to whether the eyes are open or closed. When the electrode unit 100 was used, the measurement results were almost the same as those when the vital sign information measuring electrode 200 according to Reference Example 1 was used. It was therefore found that the electrode unit 100 including the vital sign information measuring electrode 1 according to the Example can be used as an alternative to the vital sign information measuring electrode 200 according to Reference Example 1. When the electrode unit 100 including the vital sign information measuring electrode 1 according to the Example is used, it is unnecessary to apply the electrically conductive gel used for the vital sign information measuring electrode 200 according to Reference Example 1. It was therefore found that the handleability of the electrode unit 100 including the vital sign information measuring electrode 1 according to the Example is better than that of the vital sign information measuring electrode 200 according to Reference Example 1.

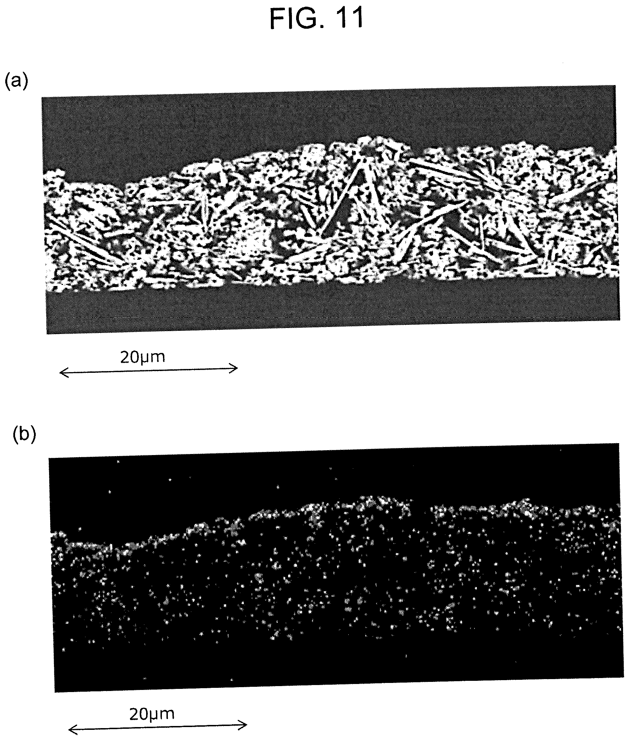

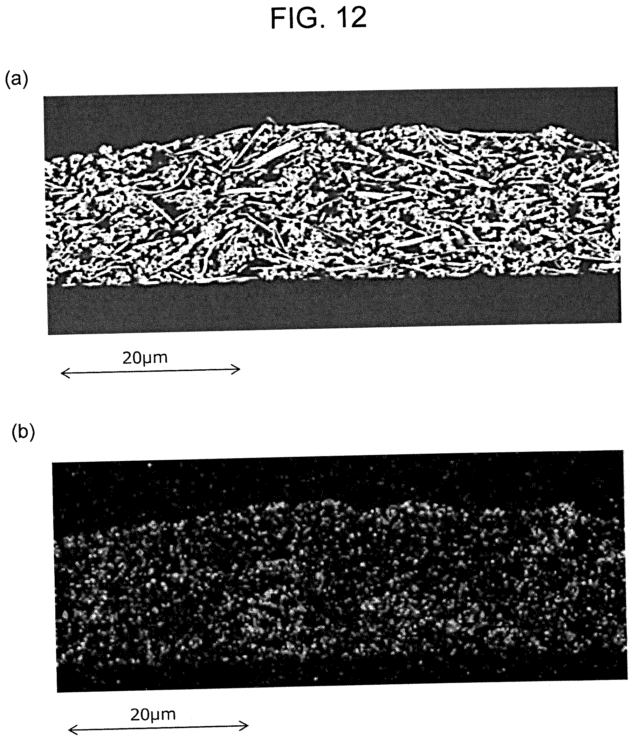

Next, the corrosion resistance of the vital sign information measuring electrode 1 according to the Example was evaluated. Specifically, the vital sign information measuring electrode 1 according to the Example and the vital sign information measuring electrode according to the Comparative Example were subjected to a sulfurization test in which they were left to stand in an atmosphere with a hydrogen sulfide concentration of 1 ppm at 40°C and a relative humidity of 80% for 96 hours. After the test, a cross section of the contact portion 21 of each of the vital sign information measuring electrodes was subjected to observation and compositional analysis using a scanning electron microscope and an analyzer included in the scanning electron microscope. The results are shown in Figs. 11 and 12. Fig. 11 shows photographs of a cross section of the vital sign information measuring electrode 1 according to the Example after the sulfurization test. Fig. 11(a) is an observation image of the cross section, and Fig. 11(b) is a compositional image of sulfur (S) in the same viewing area as Fig. 11(a). Fig. 12 shows photographs of a cross section of the vital sign information measuring electrode according to the Comparative Example after the sulfurization test. Fig. 12(a) is an observation image of the cross section, and Fig. 12(b) is a compositional image of sulfur (S) in the same viewing area as Fig. 12(a).

As can be seen from the results shown in the secondary electron images, in both the vital sign information measuring electrode 1 according to the Example (Fig. 11(a)) and the vital sign information measuring electrode according to the Comparative Example (Fig. 12(a)), the structure in which the silver powder 50 is dispersed in the synthetic resin 52 is maintained. As can be seen from the results of the compositional analysis of sulfur (S), in the vital sign information measuring electrode 1 according to the Example, sulfur (S) segregates on the surface 21S side, and the amount of sulfur (S) diffused to the inner side is small, as shown in Fig. 11(b). However, as shown in Fig. 12(b), in the vital sign information measuring electrode according to the Comparative Example, sulfur (S) is distributed substantially uniformly over the contact portion 21 at a constant concentration, and sulfurization of silver was found to procced even in the inner side of the electrode layer 20.

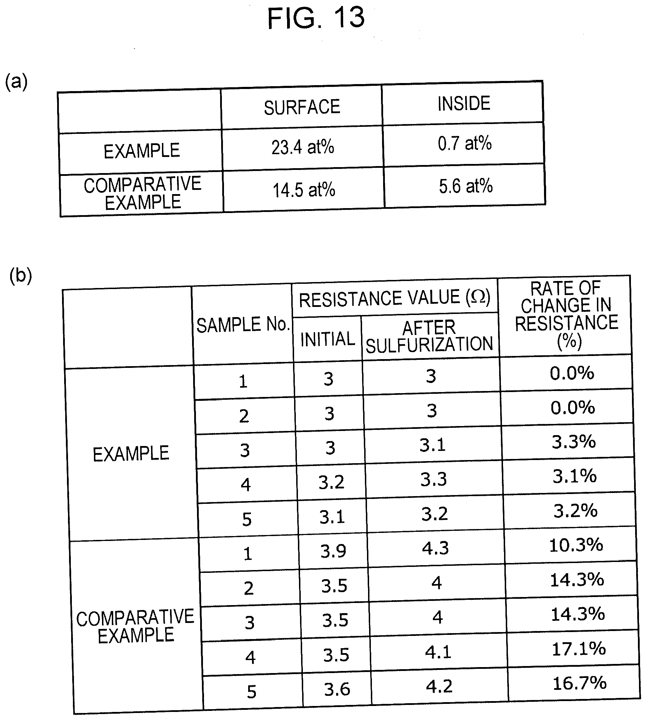

Each of the vital sign information measuring electrode 1 according to the Example and the vital sign information measuring electrode according to the Comparative Example was subjected to compositional analysis at the surface and an interior position to determine the concentration of sulfur (S) (unit: at%). The results ae shown in Fig. 13(a). As shown in Fig. 13(a), in the vital sign information measuring electrode 1 according to the Example, the concentration of sulfur (S) at the surface was 20 at% or more, but the concentration of sulfur (S) at the interior position was less than 1 at%. In the vital sign information measuring electrode according to the Comparative Example, the concentration of sulfur (S) at the surface was about 15 at%, and the concentration of sulfur (S) at the interior position was also about 5 at%. As described above, in the vital sign information measuring electrode 1 according to the Example, sulfur (S) segregates on the surface side, and diffusion of sulfur (S) toward the inner side is suppressed. However, in the vital sign information measuring electrode according to the Comparative Example, it was quantitatively found that sulfur (S) diffused from the surface side toward the inner side.

For each of the vital sign information measuring electrode 1 according to the Example and the vital sign information measuring electrode according to the Comparative Example, two measurement probes were caused to pierce through the contact portion 21 with a prescribed separation distance in an X1-X2 direction, and the resistance (unit: **Ω**) of the contact portion 21 was measured before and after the sulfurization test. The number of measurement samples was 5 for each of the vital sign information measuring electrodes. The results are shown in Fig. 13(b). As shown in Fig. 13(b), in the vital sign information measuring electrode 1 according to the Example, the resistance before the sulfurization test (the initial resistance) was about 3 **Ω**, and the rate of change in the resistance value after the sulfurization test (the rate of change in resistance, [the resistance value after the sulfurization test / the resistance value before the sulfurization test - 1]×100, unit: %) was at most about 3%. However, in the vital sign information measuring electrode according to the Comparative Example, the resistance before the sulfurization test (the initial resistance) was about 3.5 **Ω** to about 4 **Ω**, and the rate of change in resistance was at least 10% and at most 17%. Specifically, although the increase in the resistance value in the vital sign information measuring electrode 1 according to the Example after the sulfurization test was very small, the increase in the resistance value in the vital sign information measuring electrode according to the Comparative Example after the sulfurization test was evident. In the vital sign information measuring electrode according to the Comparative Example, sulfide was formed in the silver region 21C of the contact portion 21 during the sulfurization test, and the influence of the sulfide was observed as the clear increase in the resistance value.

As can be seen from these results, in the vital sign information measuring electrode 1 according to the Example, the silver chloride region 21A functions as a protective region for protecting sulfurization of silver (Ag) inside the contact portion 21.

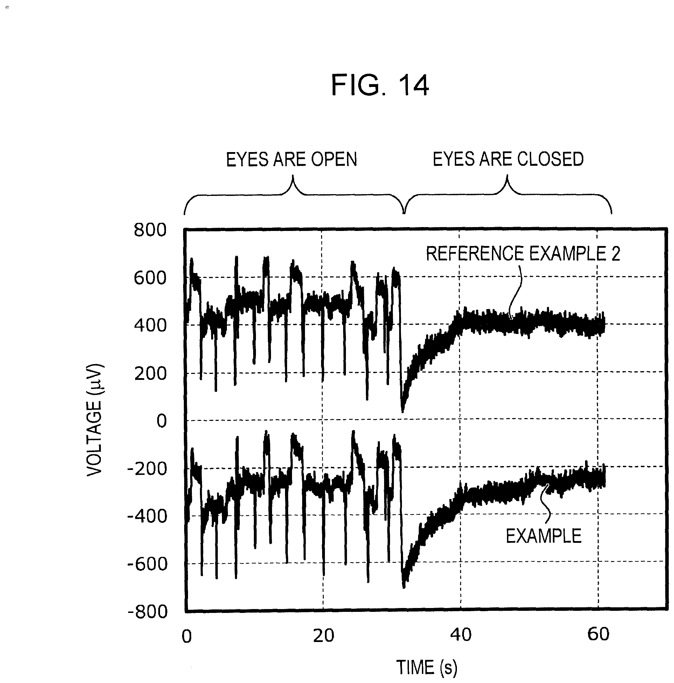

Next, the vital sign information measuring electrode 1 according to the Example after the sulfurization test was used to form the electrode unit 100 shown in Fig. 2(c), and the electrode unit 100 was fixed to the forehead of a human. Then vital sign information was measured with an earlobe grounded. For the purpose of comparison, vital sign information was measured similarly using the vital sign information measuring electrode 300 according to Reference Example 2 instead of the electrode unit 100. Before the measurement, an electrically conductive gel was applied between the vital sign information measuring electrode 300 and the forehead of the human. The results of the measurement are shown in Fig. 14.

As shown in Fig. 14, when the electrode unit 100 using the vital sign information measuring electrode 1 according to the Example after the sulfurization test was used, the results of the measurement were approximately the same as the results of the measurement using the vital sign information measuring electrode 300 according to Reference Example 2, although no electrically conductive gel was used. It was therefore found that the electrode unit 100 including the vital sign information measuring electrode 1 according to the Example is an electrode unit that can measure the vital sign information without using any electrically conductive gel. Moreover, since the vital sign information measuring electrode 1 resists corrosion, it can be used repeatedly.

### Reference Signs List

1: vital sign information measuring electrode
10: substrate
10A: principal surface of substrate 10
20: electrode layer
21: contact portion
21A: silver chloride region
21B: concentration gradient region
21C: silver region
21S: surface of contact portion 21
22: wiring portion
23: external terminal
30: spacer
50: silver powder
51: silver chloride powder
52: synthetic resin
60: resin-based composition
61: uncured resin composition
65: coating
70: electrically conductive layer
71: first portion
72: second portion
80: layered body
90: treatment bath
91: electrolyte
92: electrically conductive clip
93: cathode
94: power source
100: electrode unit
200: vital sign information measuring electrode according to Reference Example 1
201: electrode portion
202: contact portion
202A: first surface of contact portion 202
203: external terminal
204: adhesive sheet
204A: adhesive surface
300: vital sign information measuring electrode according to Reference Example 2
301: electrode portion
302: base portion
303: pin
304: spherical body
305: terminal portion
DM: application device

## Claims

1. A vital sign information measuring electrode comprising: a substrate; and an electrode layer disposed on a surface of the substrate, a part of the electrode layer serving as a contact portion that can come into contact with a living body,
wherein the contact portion of the electrode layer has a dispersion structure in which silver chloride and silver or a silver alloy are dispersed in a synthetic resin, and
wherein, in the contact portion of the electrode layer, the content of silver chloride on a surface side on which the contact portion comes into contact with the living body is higher than the content of silver chloride on an inner side.

2. The vital sign information measuring electrode according to claim 1, wherein, in the contact portion of the electrode layer, the content of silver on the inner side is higher than the content of silver on the surface side.

3. The vital sign information measuring electrode according to claim 1 or 2, wherein the contact portion of the electrode layer has a concentration gradient region in which the content of silver chloride decreases gradually from the surface side toward the inner side and the content of silver increases gradually from the surface side toward the inner side.

4. The vital sign information measuring electrode according to any of claims 1 to 3, wherein the substrate is flexible.

5. The vital sign information measuring electrode according to any of claims 1 to 4, wherein, in the contact portion of the electrode layer, the silver or silver alloy is present as a silver or silver alloy powder dispersed in the synthetic resin, and
wherein the silver chloride in the contact portion is a chloride of the silver contained in the silver or silver alloy powder.

6. The vital sign information measuring electrode according to any of claims 1 to 5, wherein the contact portion of the electrode layer has, on the surface side, a protective region that prevents corrosion of the silver on the inner side through the silver chloride.

7. A method for producing a vital sign information measuring electrode including a substrate and an electrode layer disposed on a surface of the substrate,
a part of the electrode layer serving as a contact portion that can come into contact with a living body,
wherein the contact portion of the electrode layer has a dispersion structure in which silver chloride and silver or a silver alloy are dispersed in a synthetic resin,
wherein, in the contact portion of the electrode layer, the content of silver chloride on a surface side on which the contact portion comes into contact with the living body is higher than the content of silver chloride on an inner side,
the method comprising:
an application step of applying a resin-based composition containing a silver or silver alloy powder dispersed therein to the surface of the substrate to thereby obtain a coating;
an electrically conductive layer forming step of curing the coating to form an electrically conductive layer adhering to the surface of the substrate; and
a chlorination step of subjecting the electrically conductive layer to anodic electrolysis using a liquid containing chloride ions to convert part of the silver or silver alloy powder contained in the electrically conductive layer to silver chloride to thereby form the contact portion of the electrode layer from a first part of the electrically conductive layer.

8. The method for producing a vital sign information measuring electrode according to claim 7, wherein, in the chlorination step, a concentration gradient region in which the content of silver chloride decreases gradually from the surface side toward the inner side and the content of silver increases gradually from the surface side toward the inner side is formed in the contact portion.

9. The method for producing a vital sign information measuring electrode according to claim 7 or 8, wherein, in the chlorination step, a protective region that prevents corrosion of the silver on the inner side through the silver chloride is formed on the surface side of the contact portion.

10. The method for producing a vital sign information measuring electrode according to any of claims 7 to 9, wherein the substrate has a flat plate shape,
wherein the electrode layer is disposed on a first principal surface of the substrate,
wherein the electrode layer includes an external terminal for outputting an electric signal including vital sign information collected by the contact portion to the outside, and
wherein the external terminal is formed from a second part of the electrically conductive layer, the second part being not subjected to the chlorination step.

11. The method for producing a vital sign information measuring electrode according to claim 10, wherein the electrode layer further includes a wiring portion that electrically connects the contact portion to the external terminal, and
wherein the wiring portion is formed from a third part of the electrically conductive layer formed in the electrically conductive layer forming step.

12. The method for producing a vital sign information measuring electrode according to claim 11, wherein, in chlorination step, the third part of the electrically conductive layer is subjected to anodic electrolysis so that the wiring portion includes an electric conduction region based on the electrically conductive layer and a protective region formed on the surface side of the electric conduction region and containing the silver chloride, the third part of the electrically conductive layer corresponding to the wiring portion.
